(19) Europäisches Patentamt
European Patent Office
Office européen des brevets

(11) **EP 3 250 979 B1**

(12) **EUROPÄISCHE PATENTSCHRIFT**

(45) Veröffentlichungstag und Bekanntmachung des Hinweises auf die Patenterteilung:
**01.01.2020 Patentblatt 2020/01**

(21) Anmeldenummer: **15801149.4**

(22) Anmeldetag: **25.11.2015**

(51) Int Cl.:
***G01D 11/24*** *(2006.01)*

(86) Internationale Anmeldenummer:
**PCT/EP2015/077635**

(87) Internationale Veröffentlichungsnummer:
**WO 2016/119945 (04.08.2016 Gazette 2016/31)**

(54) **SENSOR**

SENSOR

CAPTEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.01.2015 DE 102015101112**

(43) Veröffentlichungstag der Anmeldung:
**06.12.2017 Patentblatt 2017/49**

(73) Patentinhaber: **TDK Corporation Tokyo 108-0023 (JP)**

(72) Erfinder:
• **SCHOBER, Armin**
  **81667 München (DE)**
• **KUIVALAINEN, Jani**
  **98052 Washington (DE)**

(74) Vertreter: **Epping - Hermann - Fischer Patentanwaltsgesellschaft mbH Schloßschmidstraße 5 80639 München (DE)**

(56) Entgegenhaltungen:
**WO-A1-2006/096005     US-A1- 2010 328 059**

EP 3 250 979 B1

**Beschreibung**

Es wird ein Sensor, insbesondere ein Umgebungssensor, angegeben.

Sensoren, insbesondere Umgebungssensoren, kommen zunehmend im Bereich tragbarer elektronischer Konsumgüter wie in Mobiltelefonen, Armbanduhren und Armbändern zum Einsatz. Besonders bei einer Verwendung im Sportbereich wird erwartet, dass der Sensor wasserdicht ist.

Die Patentdokumente US 6,754,137 B1, US 5,500,835 A, DE 10 2006 056 128 B4 und EP 0 640 896 B1 beschreiben jeweils eine Armbanduhr mit einem Drucksensor. Zum Schutz des Sensorelements vor eindringendem Wasser kann das Sensorelement von einem wasserabweisenden Gel umgeben sein oder durch eine Membran von der Umgebungsluft getrennt sein. Aus der Patentanmeldung DE 10 2006 040 665 A1 geht ein Drucksensor für ein Fahrzeug hervor, bei dem ein Eindringen von Wasser in ein Gehäuse des Sensors durch einen wasserundurchlässigen Filter verhindert wird. Derartige Sensoren haben oft eine große Baugröße und/oder sind teuer in der Herstellung.

Die Patentanmeldung US 2010/0328059 A1 betrifft eine Vorrichtung zur Zustandsbestimmung eines Reifens, aufweisend einen Drucksensor, bei dem ein Eindringen von Wasser in eine Luftzufuhr durch die Oberflächenspannung des Wassers verhindert wird. Die Patentanmeldung WO 2006/096005 A1 betrifft ein wasserundurchlässiges Substrat, bei dem ein Eindringen von Wasser durch ein hydrophobes Material und durch eine Mikrostrukturierung verhindert wird.

Es ist eine Aufgabe der vorliegenden Erfindung, einen verbesserten Sensor anzugeben. Diese Aufgabe wird durch einen Sensor nach Anspruch 1 gelöst.

Es wird ein Sensor aufweisend eine Kammer mit einem darin angeordneten Sensorelement angegeben, wobei die Kammer ein erstes Volumen an Luft aufweist. Der Sensor weist eine röhrenförmige Luftzufuhr zur Kammer auf, wobei die Luftzufuhr ein zweites Volumen an Luft aufweist. Vorzugsweise ist die Kammer abgesehen von der röhrenförmigen Luftzufuhr luftdicht abgeschlossen. Somit kann ein Luftaustausch der Kammer nur mit der Luftzufuhr erfolgen. Es kann jedoch anderweitig keine Luft aus der Kammer entweichen oder eindringen. Ein Vordringen von Wasser durch die Luftzufuhr zum Sensorelement wird durch die das zweite Volumen definierenden Abmessungen der Luftzufuhr verhindert. Dabei ist das Vordringen von Wasser mindestens bis zu einem Außendruck verhindert, der kleiner oder gleich einem vorgegebenen maximalen Außendruck ist. Statt dem maximalen Außendruck kann auch eine maximale Wassertiefe vorgegeben sein.

Bei einem derartigen Sensor kann somit ein Vordringen von Wasser zum Sensorelement durch die Abmessungen der Luftzufuhr verhindert werden, ohne dass zusätzliche Barriereelemente zum Schutz gegen ein Eindringen von Wasser benötigt werden. Insbesondere wird kein Barriereelement benötigt, das in einem Verbindungsweg zwischen dem Außenraum und dem Sensorelement angeordnet ist. Der Verbindungsweg führt insbesondere durch das zweite Volumen der Luftzufuhr und das erste Volumen der Kammer hindurch. Ein Sensor, bei dem kein derartiges Barriereelement vorhanden ist, ist besonders kostengünstig herstellbar. Weiterhin ist eine Miniaturisierung des Sensors ermöglicht.

Beispielsweise weist der Sensor keine wasserdichte Membran auf, die das Sensorelement vom Außenraum des Sensors trennt. Es ist auch kein gelförmiges Barriereelement nötig, das das Sensorelement beispielsweise umgibt. Somit kann die Umgebungsluft direkt auf das Sensorelement einwirken, so dass eine besonders genaue Messung ermöglicht ist.

Beispielsweise handelt es sich um einen Sensor zur Messung von Eigenschaften der Außenumgebung, beispielsweise der Umgebungsluft. Ein derartiger Umgebungssensor ist beispielsweise als Drucksensor, insbesondere als barometrischer Drucksensor, als Luftfeuchtigkeitssensor, insbesondere als Sensor zur Messung der relativen Luftfeuchtigkeit, oder als Gassensor ausgebildet. Ein Gassensor ist zur Messung der Konzentration eines Gases, beispielsweise Kohlendioxid, Kohlenmonoxid oder Ozon ausgebildet. Bei derartigen Sensoren kann eine direkte Wechselwirkung der Umgebungsluft mit dem Sensorelement für die Messung von Vorteil oder sogar notwendig sein.

Der Sensor ist wasserdicht in dem Sinne ausgebildet, dass Wasser nicht bis zu wasserempfindlichen Komponenten des Sensors vordringen kann. Dabei kann der Sensor neben dem Sensorelement noch weitere wasserempfindliche Komponenten, insbesondere elektrische Komponenten aufweisen. Die weiteren Komponenten sind beispielsweise von einem wasserdichten Gehäuse umgeben.

Insbesondere wird durch die Abmessungen der Luftzufuhr nicht nur ein Vordringen von Wasser zum Sensorelement, sondern ein Eindringen von Wasser in die Kammer verhindert.

Insbesondere sind die Abmessungen der Luftzufuhr derart gewählt, dass die Luft, die sich bei atmosphärischen Luftdruck auf Meereshöhe in der Kammer und in der Luftzufuhr befindet, bei einer Erhöhung des Außendrucks ein Volumen nicht kleiner als das erste Volumen annimmt. Das Volumen der Luft bei atmosphärischem Luftdruck ist die Summe aus dem ersten und dem zweiten Volumen. Bei einem Eintauchen des Sensors in Wasser wird die innerhalb der Kammer und der Luftzufuhr befindliche Luft soweit komprimiert, bis der Druck der Luft dem durch das Wasser einwirkenden Außendruck entspricht. Da das Volumen der Luft mindestens so groß ist wie das erste Volumen innerhalb der Kammer, ist das erste Volumen vollständig mit Luft gefüllt. Somit kann das in die Luftzufuhr eindringende Wasser nicht bis in die Kammer hinein gelangen.

Geeignete Abmessungen der Luftzufuhr lassen sich aus dem idealen Gasgesetz ableiten. Bei einem at-

mosphärischen Luftdruck $p_{at}$ und einem vorgegebenen maximalen Außendruck $p_{max}$, bei dem noch kein Wasser in die Kammer eindringen soll, ergibt sich folgende Bedingung für das Verhältnis des zweiten Volumens $V_2$ zum ersten Volumen $V_1$:

$$V_2/V_1 \; >= \; p_{max}/p_{at} \; - \; 1$$

Setzt man für den Luftdruck auf Meereshöhe 1 bar und für die Zunahme des Drucks unter Wasser 1 bar pro 10 m Wassertiefe an, so ergibt sich bei Normalbedingungen (Druck von 1 bar und Temperatur von 20 °C) folgende Bedingung für die Volumenverhältnisse bei einer vorgegebenen maximalen Wassertiefe $d_{max}$ in Metern:

$$V_2/V_1 \; >= \; 0,1 \; m^{-1} \; \cdot \; d_{max}$$

Somit muss beispielsweise bei einer vorgegebenen maximalen Wassertiefe von 10 m das zweite Volumen mindestens so groß sein wie das erste Volumen, was erfindungsgemäß der Fall ist. In einer Ausführungsform ist das zweite Volumen mindestens doppelt so groß wie das erste Volumen. In diesem Fall ist ein Vordringen von Wasser zum Sensorelement mindestens bis zu einer Wassertiefe von 20 m verhindert. Entsprechend erstreckt sich ein Arbeitsdruckbereich, in dem ein Vordringen von Wasser zum Sensorelement verhindert wird, beispielsweise mindestens über den Bereich von 1 bar bis 2 bar Außendruck, vorzugsweise mindestens über den Bereich von 1 bar bis 3 bar Außendruck.

In einer Ausführungsform ist die Kammer quaderförmig ausgebildet. Die Kammer weist beispielsweise eine Öffnung für die Luftzufuhr auf. Vorzugsweise ist dies die einzige Öffnung der Kammer. Vorzugsweise führt ein Verbindungsweg vom Sensorelement zum Außenraum lediglich durch die Kammer und die Luftzufuhr.

Erfindungsgemäß weist die Luftzufuhr einen maximalen Innendurchmesser auf, der derart gering ist, dass die eingeschlossene Luft bei einem Eintauchen in Wasser nicht durch die Luftzufuhr nach außen entweichen kann. Der maximale Innendurchmesser der Luftzufuhr ist vorzugsweise wesentlich kleiner als die Ausdehnung der Kammer senkrecht zur Einströmrichtung der Luft. Beispielsweise ist der Innendurchmesser kleiner oder gleich 1 mm. Die Länge der Luftzufuhr ist erfindungsgemäß wesentlich größer als ein Innendurchmesser der Luftzufuhr. Die Luftzufuhr weist beispielsweise eine Länge von mindestens 5 mm auf.

In einer Ausführungsform ist die Luftzufuhr als separates Element ausgebildet. Insbesondere ist die Luftzufuhr separat von einem Gehäuse der Kammer ausgebildet und/oder separat von einem äußeren Gehäuse des Sensors ausgebildet. Beispielsweise ist die Luftzufuhr als separater Schlauch ausgebildet.

In einer Ausführungsform ist die Luftzufuhr aus einem flexiblen Material gebildet. Dies ermöglicht es, die Luftzufuhr beispielsweise beim Einbau in ein Gehäuse zu biegen, so dass eine Anpassung an die geometrischen Abmessungen des Sensors, insbesondere eines äußeren Gehäuses ermöglicht ist. Beispielsweise weist das Material der Luftzufuhr Silikon auf.

In einer Ausführungsform ist die Luftzufuhr in ein Gehäuse des Sensors integriert. Insbesondere kann die Luftzufuhr einteilig mit einem Gehäuse ausgebildet sein. Beispielsweise ist ein Gehäuse in einem Spritzgussverfahren hergestellt, wobei die Luftzufuhr beim Spritzgießen ausgebildet wird. Insbesondere kann die Luftzufuhr beim Spritzgießen als Durchführung durch das Gehäuse gebildet werden. Dies ermöglicht eine besonders kostengünstige Herstellung der Luftzufuhr.

In einer Ausführungsform weist die Luftzufuhr wenigstens eine Knick oder eine Biegung auf. Auf diese Weise können die erforderlichen Abmessungen des Sensors gering gehalten werden.

Der Sensor ist beispielsweise als barometrischer Drucksensor ausgebildet. Das Sensorelement ist beispielsweise als piezoresistives oder kapazitives Sensorelement ausgebildet.

Vorzugsweise ist das Sensorelement miniaturisiert ausgebildet. Insbesondere weist das Sensorelement Abmessungen von wenigen Millimetern oder kleiner auf. Dies ermöglicht es, das erste Volumen besonders gering zu halten. In diesem Fall kann auch das zweite Volumen und insbesondere auch die Länge der Luftzufuhr besonders gering gehalten werden.

Somit kann der Sensor insgesamt miniaturisiert ausgebildet sein. Beispielsweise ist er für eine Verwendung in einem Mobiltelefon, einer Armbanduhr oder einem Armband geeignet.

Im Folgenden werden die hier beschriebenen Gegenstände anhand von schematischen und nicht maßstabsgetreuen Ausführungsbeispielen näher erläutert.

Es zeigen:

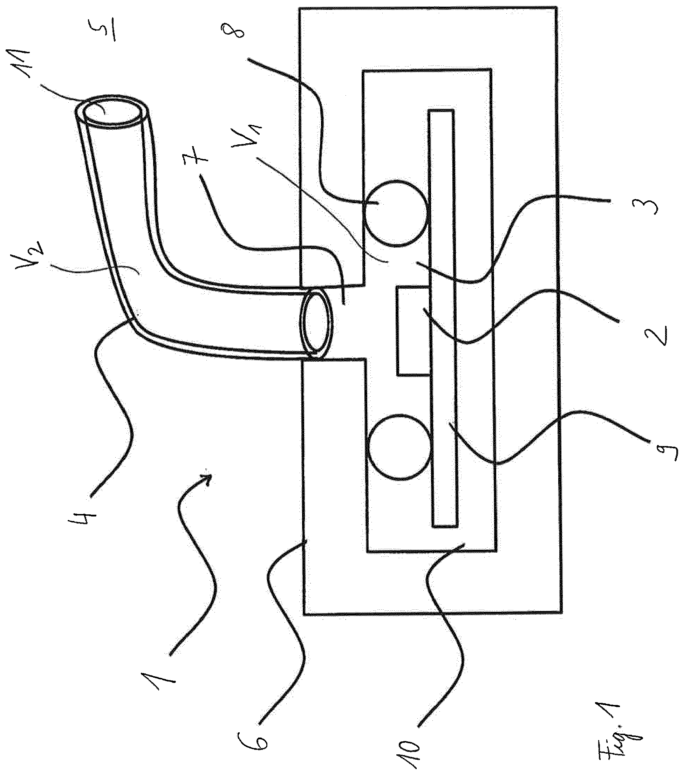

Figur 1 in schematischer Schnittansicht einen Sensor gemäß einer Ausführungsform.

Figur 1 zeigt einen Sensor 1, der beispielsweise als Drucksensor ausgebildet ist.

Der Sensor 1 weist ein Sensorelement 2 auf, das in einer Kammer 3 angeordnet ist. Innerhalb der Kammer 3 befindet sich ein erstes Volumen $V_1$ an Luft, welches das Sensorelement 2 teilweise umgibt. Insbesondere ist das Sensorelement 2 seitlich und nach oben hin vom ersten Volumen $V_1$ umgeben. Das erste Volumen $V_1$ ist durch eine röhrenförmige Luftzufuhr 4 mit einem Außenraum 5 verbunden. Der Sensor 1 weist eine offene Struktur auf, so dass von außen Luft und Wasser in die Luftzufuhr 4 eindringen kann.

Die Kammer 3 ist teilweise von einem Gehäuse 6 begrenzt. Das Gehäuse 6 weist eine Öffnung 7 auf, von der die Luftzufuhr 4 wegführt. In der gezeigten Ausführungsform ist die Luftzufuhr 4 teilweise in die Öffnung 7 eingeführt. Die Luftzufuhr 4 kann beispielsweise auch vollständig in die Öffnung 7 eingeführt sein oder sich außen an die Öffnung 7 anschließen. Seitlich ist die Kammer 3 von einem Dichtelement 8 begrenzt, das beispielsweise als Dichtring ausgebildet ist. Das Dichtelement 8 dichtet die Kammer 3 luftdicht ab. Das Sensorelement 2 ist auf einem Träger 9, insbesondere einer Leiterplatte, angeordnet. Der Träger 9 begrenzt die Kammer 3 nach unten.

Das Gehäuse 6 kann noch weitere Komponenten des Sensors 1 umschließen. Beispielsweise sind in einem weiteren Innenraum 10, der von der Kammer 3 durch das Dichtelement 8 luftdicht abgedichtet ist, weitere Komponenten, insbesondere elektronische Komponenten angeordnet. Insbesondere ist das luftgefüllte Volumen $V_1$ luftdicht vom weiteren Innenraum 10 getrennt. Dabei ist das Dichtelement 8 mindestens im Arbeitsdruckbereich luftdicht, insbesondere bei einem Außendruck in einem Bereich zwischen atmosphärischem Luftdruck und einem vorgegebenen Maximaldruck. Somit ist ein Luftfluss aus dem Volumen $V_1$ lediglich in die Luftzufuhr 4, aber nicht in andere Bereiche des Sensors 1 möglich. Der weitere Innenraum 10 ist vorzugsweise luftdicht und wasserdicht vom Außenraum 5 abgeschlossen. Somit ist der Luftdruck im weiteren Innenraum 10 vorzugsweise konstant, beispielsweise immer bei Normaldruck.

Das erste Volumen $V_1$ ist über das zweite Volumen $V_2$ luftdurchlässig mit dem Außenraum 5 verbunden. Die Luftzufuhr 4 ist dazu ausgebildet, dass bei einem Eintauchen des Sensors 1 in Wasser das Wasser in die Luftzufuhr 4 über eine äußere Öffnung 11 eindringen kann. Da die Luftzufuhr 4 abgesehen von der äußeren Öffnung 11 nur mit der Kammer 3 luftdurchlässig verbunden ist und die Kammer 3 abgesehen von der Luftzufuhr 4 luftdicht abgeschlossen ist, kann die in der Luftzufuhr 4 und der Kammer 3 eingeschlossene Luft nicht entweichen. Somit wird die innerhalb der Luftzufuhr 4 und der Kammer 3 befindliche Luft soweit komprimiert, bis der Druck der Luft dem Außendruck entspricht. Die Luftzufuhr 4 weist derartige Abmessungen auf, dass bei einem äußeren Druck, der kleiner oder gleich einem vorgegebenen Maximaldruck ist, das eindringende Wasser nicht bis in die Kammer 3 gelangen kann.

Der abgebildete Sensor 1 ist beispielsweise mindestens bis zu einer Tiefe $d_{max} = 20$ m wasserdicht. Gemäß der Bedingung $V_2/V_1 \geq 0,1$ m$^{-1} \cdot d_{max}$ ist das zweite Volumen mindestens doppelt so groß wie das erste Volumen. Oftmals ist eine Wasserdichte bis zu einer Tiefe von 50 m gewünscht. In diesem Fall ist das zweite Volumen mindestens fünf Mal so groß wie das erste Volumen.

Beispielsweise weist das Sensorelement 2 eine Abmessung von 2 mm x 2 mm x 0,8 mm (Breite x Länge x Höhe) auf. Die Kammer 3 wird beispielsweise von einem Dichtelement 8 in Form eines Dichtrings nach außen begrenzt und weist beispielsweise einen lateralen Durchmesser von 2,5 mm und eine Höhe von 1 mm auf. Das erste Volumen $V_1$ innerhalb der Kammer 3 berechnet sich aus der Differenz des Kammervolumens und des vom Sensorelement eingenommenen Volumens zu 1,7 mm$^3$. Bei einer vorgegebenen maximalen Wassertiefe von 20 m ergibt sich ein zweites Volumen $V_2$ von mindestens 3,4 mm$^3$. Somit müsste bei einem Innendurchmesser der Luftzufuhr 4 von 0,8 mm die Luftzufuhr 4 eine Länge von mindestens 6,8 mm aufweisen.

Die Luftzufuhr 4 weist einen gebogenen Verlauf auf. Die Luftzufuhr 4 ist vorzugsweise innerhalb eines äußeren Gehäuses (nicht abgebildet) des Sensors 1 angeordnet. Insbesondere ragt die Luftzufuhr 4 nicht aus dem äußeren Gehäuse heraus. Das äußere Gehäuse kann auch einteilig mit dem Gehäuse 6 ausgebildet sein. Beispielsweise reicht das Gehäuse bis zur Öffnung der Luftzufuhr 4 zum Außenraum 5. Das äußere Gehäuse kann aber auch als separates Bauteil zum Gehäuse 6 ausgebildet sein.

In einer Ausführungsform ist die Luftzufuhr 4 als separates Element ausgebildet. Beispielsweise ist die Luftzufuhr 4 durch ein flexibles Material gebildet. Beispielsweise weist das Material Silikon auf. In einer alternativen Ausführungsform ist die Luftzufuhr 4 in ein äußeres Gehäuse integriert. Beispielsweise ist das äußere Gehäuse spritzgegossen. Die Luftzufuhr 4 kann im Spritzgussverfahren als Durchführung durch das Gehäuse ausgebildet sein.

Bezugszeichenliste

1    Sensor
2    Sensorelement
3    Kammer
4    Luftzufuhr
5    Außenraum
6    Gehäuse
7    Öffnung
8    Dichtelement
9    Träger
10   weiterer Innenraum
11   äußere Öffnung

$V_1$   erstes Volumen
$V_2$   zweites Volumen

**Patentansprüche**

1.   Sensor,
aufweisend eine Kammer (3) mit einem darin angeordneten Sensorelement (2), wobei die Kammer (3) ein erstes Volumen ($V_1$) an Luft aufweist, und aufweisend eine Luftzufuhr (4) zur Kammer (3), wobei die Luftzufuhr (4) ein zweites Volumen ($V_2$) an Luft aufweist, wobei ein Vordringen von Wasser durch die Luftzufuhr (4) zum Sensorelement (2) durch die das zweite Volumen ($V_2$) definierenden Abmessungen der Luftzufuhr (4) verhindert wird, wobei die Luftzufuhr (4) einen maximalen Innendurchmesser aufweist, der derart gering ist, dass die eingeschlossene Luft bei einem Eintauchen in Wasser nicht durch die Luftzufuhr (4) nach außen entweicht, wobei die Länge der Luftzufuhr (4) wesentlich größer ist als der Innendurchmesser der Luftzufuhr (4), **dadurch gekennzeichnet, dass** die Luftzufuhr (4) eine röhrenförmige Luftzufuhr (4) ist, und dass das zweite Volumen ($V_2$) mindestens so groß ist wie das erste Volumen ($V_1$).

2. Sensor nach Anspruch 1, bei dem bei einem Eintauchen des Sensors (1) in Wasser und einem Eindringen von Wasser in die Luftzufuhr (4) das Gesamtvolumen der Luft in der Kammer (3) und in der Luftzufuhr (4) komprimiert wird, ohne dass die Luft entweicht, wobei das Gesamtvolumen der komprimierten Luft mindestens so groß ist wie das Volumen ($V_1$) der Kammer (3), so dass die Kammer (3) vollständig mit Luft gefüllt bleibt.

3. Sensor nach einem der vorhergehenden Ansprüche, bei dem der maximale Innendurchmesser der Luftzufuhr (4) wesentlich kleiner ist als die Ausdehnung der Kammer (3) senkrecht zur Einströmrichtung der Luft.

4. Sensor nach einem der vorhergehenden Ansprüche, bei dem ein Vordringen von Wasser zum Sensorelement (2) mindestens bis zu einer vorgegebenen maximalen Wassertiefe $d_{max}$ in Metern verhindert wird, wobei für das Verhältnis des zweiten Volumens ($V_2$) zum ersten Volumen ($V_1$) folgende Bedingung gilt

$$V_2/V_1 \geq 0,1 \ m^{-1} \cdot d_{max}.$$

5. Sensor nach einem der vorhergehenden Ansprüche, bei dem ein Vordringen von Wasser zum Sensorelement (2) mindestens bis zu einer Wassertiefe von 20 m verhindert wird.

6. Sensor nach einem der vorhergehenden Ansprüche, der frei von einem Barriereelement zum Schutz gegen ein Vordringen von Wasser zum Sensorelement (2) ist.

7. Sensor nach Anspruch 6, der frei von einem als Membran ausgebildeten Barriereelement ist.

8. Sensor nach einem der Ansprüche 6 oder 7, der frei von einem als Gel ausgebildeten Barriereelement ist.

9. Sensor nach einem der vorhergehenden Ansprüche, bei dem die Luftzufuhr (4) aus einem flexiblen Material gebildet ist.

10. Sensor nach einem der vorhergehenden Ansprüche, bei dem die Luftzufuhr (4) in ein Gehäuse (6) integriert ist und bei dem das Gehäuse (6) spritzgegossen ist, wobei die Luftzufuhr beim Spritzgießen ausgebildet wird.

11. Sensor nach einem der vorhergehenden Ansprüche, bei dem die Luftzufuhr (4) wenigstens einen Knick oder eine Biegung aufweist.

12. Sensor nach einem der vorhergehenden Ansprüche, bei dem das Sensorelement (2) seitlich und nach oben hin vom ersten Volumen ($V_1$) umgeben ist.

13. Sensor nach einem der vorhergehenden Ansprüche, der zu einer Verwendung in einem Mobiltelefon, einer Armbanduhr oder einem Armband geeignet ist.

14. Sensor nach einem der vorhergehenden Ansprüche, der als Drucksensor, als Luftfeuchtigkeitssensor oder als Gassensor ausgebildet ist.

**Claims**

1. Sensor, having a chamber (3) with a sensor element (2) arranged therein, wherein the chamber (3) has a first volume ($V_1$) of air, and having an air supply (4) to the chamber (3), wherein the air supply (4) has a second volume ($V_2$) of air, wherein penetration of water through the air supply (4) to the sensor element (2) is prevented by the dimensions of the air supply (4) that define the second volume ($V_2$), wherein the air supply (4) has a maximum inside diameter that is small enough that the trapped air during immersion in water does not escape to the outside through the air supply (4), wherein the length of the air supply (4) is much greater than the inside diameter of the air supply (4), **characterized in that** the air supply (4) is a tubular air supply (4), and **in that** the second volume ($V_2$) is at least as great as the first volume ($V_1$).

2. Sensor according to Claim 1, in the case of which, when the sensor (1) is immersed in water and water penetrates into the air supply (4), the overall volume of air in the chamber (3) and in the air supply (4) is compressed without the air escaping, wherein the overall volume of the compressed air is at least as great as the volume ($V_1$) of the chamber (3), so that the chamber (3) remains completely filled with air.

3. Sensor according to one of the preceding claims, in the case of which the maximum inside diameter of the air supply (4) is much smaller than the extent of the chamber (3) perpendicularly to the direction of inflow of the air.

4. Sensor according to one of the preceding claims, in the case of which penetration of water to the sensor element (2) is prevented at least to a prescribed maximum water depth $d_{max}$ in metres, wherein the following condition applies to the ratio of the second volume ($V_2$) to the first volume ($V_1$)

$$V_2/V_1 \geq 0.1 \ m^{-1} \cdot d_{max}.$$

5. Sensor according to one of the preceding claims, in the case of which penetration of water to the sensor element (2) is prevented at least to a prescribed water depth of 20 m.

6. Sensor according to one of the preceding claims, which does not have a barrier element for protection against penetration of water to the sensor element (2).

7. Sensor according to Claim 6, which does not have a barrier element formed as a membrane.

8. Sensor according to either of Claims 6 and 7, which does not have a barrier element formed as a gel.

9. Sensor according to one of the preceding claims, in the case of which the air supply (4) is formed from a flexible material.

10. Sensor according to one of the preceding claims, in the case of which the air supply (4) is integrated in a housing (6) and in the case of which the housing (6) is injection-moulded, wherein the air supply is formed during the injection moulding.

11. Sensor according to one of the preceding claims, in the case of which the air supply (4) has at least one kink or bend.

12. Sensor according to one of the preceding claims, in the case of which the sensor element (2) is surrounded laterally and upwardly by the first volume ($V_1$).

13. Sensor according to one of the preceding claims, which is suitable for use in a mobile phone, a wristwatch or an armband.

14. Sensor according to one of the preceding claims, which is designed as a pressure sensor, as an air humidity sensor or as a gas sensor.

**Revendications**

1. Capteur,
comportant un compartiment (3) doté d'un élément de capteur (2) agencé dans celui-ci, dans lequel le compartiment (3) comporte un premier volume d'air ($V_1$), et
comportant une alimentation en air (4) dans le compartiment (3), dans lequel l'alimentation en air (4) comporte un deuxième volume d'air ($V_2$),
dans lequel une pénétration d'eau par l'alimentation en air (4) de l'élément de capteur (2) est prévenue par les dimensions de l'alimentation en air (4) qui définissent le deuxième volume ($V_2$),
dans lequel l'alimentation en air (4) possède un diamètre intérieur maximal, lequel est réduit de telle sorte que l'air piégé lors d'une immersion dans l'eau ne s'échappe pas vers l'extérieur à travers l'alimentation en air (4),
dans lequel la longueur de l'alimentation en air (4) est sensiblement supérieure au diamètre intérieur de l'alimentation en air (4),
**caractérisé en ce que** l'alimentation en air (4) est une alimentation en air tubulaire (4), et **en ce que** le deuxième volume ($V_2$) est supérieur ou égal au premier volume ($V_1$).

2. Capteur selon la revendication 1,
où, lors d'une immersion du capteur (1) dans l'eau et d'une infiltration d'eau dans l'alimentation en air (4), le volume total de l'air dans le compartiment (3) et dans l'alimentation en air (4) est comprimé, sans que l'air ne s'échappe,
dans lequel le volume total de l'air comprimé est supérieur ou égal au volume ($V_1$) du compartiment (3), de sorte que le compartiment (3) reste entièrement rempli d'air.

3. Capteur selon l'une des revendications précédentes,
où le diamètre intérieur maximal de l'alimentation en air (4) est sensiblement inférieur à l'étendue du compartiment (3) perpendiculairement à la direction d'afflux de l'air.

4. Capteur selon l'une des revendications précédentes,
où une pénétration d'eau dans l'élément capteur (2) est prévenue jusqu'à une profondeur d'eau maximale prédéfinie $d_{max}$ en mètres,
dans lequel la condition suivante s'applique pour le ratio du deuxième volume (V$_2$) au premier volume (V$_1$)

gaz.

$$V_2/V_1 \geq 0{,}1m^{-1} \cdot d_{max}.$$

**5.** Capteur selon l'une des revendications précédentes,
où une pénétration d'eau dans l'élément capteur (2) est prévenue jusqu'à une profondeur d'eau de 20 m.

**6.** Capteur selon l'une des revendications précédentes,
lequel est dépourvu d'un élément de barrière pour la protection contre la pénétration d'eau dans l'élément capteur (2).

**7.** Capteur selon la revendication 6,
lequel est dépourvu d'un élément de barrière réalisé comme membrane.

**8.** Capteur selon l'une des revendications 6 ou 7,
lequel est dépourvu d'un élément de barrière réalisé comme gel.

**9.** Capteur selon l'une des revendications précédentes,
où l'alimentation en air (4) est formée d'un matériau flexible.

**10.** Capteur selon l'une des revendications précédentes,
où l'alimentation en air (4) est intégrée dans un boîtier (6) et où le boîtier (6) est moulé par injection, dans lequel l'alimentation en air est réalisée lors d'un moulage par injection.

**11.** Capteur selon l'une des revendications précédentes,
où l'alimentation en air (4) comporte au moins un coude ou une courbure.

**12.** Capteur selon l'une des revendications précédentes,
où l'élément de capteur (2) est entouré latéralement et vers le haut par le premier volume (V$_1$).

**13.** Capteur selon l'une des revendications précédentes,
lequel est adapté à une utilisation dans un téléphone mobile, dans une montre-bracelet ou dans un bracelet.

**14.** Capteur selon l'une des revendications précédentes,
lequel est réalisé comme capteur de pression, comme capteur d'humidité de l'air ou comme capteur de

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6754137 B1
- US 5500835 A
- DE 102006056128 B4
- EP 0640896 B1
- DE 102006040665 A1
- US 20100328059 A1
- WO 2006096005 A1